# FASCICULE DE BREVET EUROPEEN

(11) **EP 1 090 632 B1**
(45) Date de publication et mention de la délivrance du brevet: **04.02.2004**
(21) Numéro de dépôt: 00402657.1
(22) Date de dépôt: 26.09.2000
(51) Int. Cl.: A61K 7/50, A61K 7/06

(54) **Composition de lavage des matières kératiniques, à base d'un agent tensio-actif détergent, d'un polyorganosiloxane, d'un polymère cationique et d'un terpolymère acrylique**
Zusammensetzung zur Reinigung von Keratinsubstanzen auf der Basis eines reini- genden grenzflächenaktiven Stoffes, eines Polyorganosiloxans, eines kationischenPolymers und eines Acrylterpolymers.
A washing composition for keratinous materials based on a surfactant, a polyorganosiloxane, a cationic polymer and an acrylic terpolymer

(30) Priorité: 29.09.1999 FR 9912164
(43) Date de publication de la demande: 11.04.2001
(73) Titulaire: L'OREAL, 75008 Paris (FR)
(72) Inventeur: Maurin, Véronique, 75016 Paris (FR); Beauquey, Bernard, 92110 Clichy (FR)
(74) Mandataire: Casalonga, Axel

(56) Documents cités:
- EP-A- 0 824 914
- EP-A- 0 825 200
- WO-A-92/10162
- WO-A-94/06403
- US-A- 5 853 707

## Description

La présente invention concerne d'une manière générale des compositions de lavage des matières kératiniques, à base d'un agent tensio-actif détergent, d'un polyorganosiloxane de viscosité spécifique, d'un polymère cationique et d'un terpolymère acrylique, ainsi qu'un procédé de lavage mettant en oeuvre ces compositions.

Les polyorganosiloxanes sont généralement utilisés dans les compositions de shampooings en tant qu'agents conditionneurs pour améliorer la douceur, le toucher et le démêlage des cheveux. Cependant, on a constaté que ces polyorganosiloxanes conduisaient à la formation d'une couche inesthétique à la surface du shampooing, indésirable pour le consommateur. Pour éviter l'apparition de ce phénomène, des agents de stabilisation tels que les polymères acryliques réticulés du type Carbopol sont fréquemment utilisés. Néanmoins, ces agents de stabilisation présentent l'inconvénient de diminuer les performances cosmétiques des shampooings, notamment en rendant les cheveux plus chargés et plus rêches.

Il était donc nécessaire de mettre au point une composition cosmétique détergente contenant des polyorganosiloxanes, en particulier un shampooing, qui ait un aspect esthétique satisfaisant tout en conférant des performances cosmétiques acceptables aux matières kératiniques, à savoir notamment les cheveux et le cuir chevelu.

La demanderesse a découvert, de façon surprenante, qu'il était possible de formuler des compositions de lavage des matières kératiniques, notamment des shampooings, ayant les propriétés recherchées, en utilisant dans ces compositions un agent tensio-actif détergent, un polyorganosiloxane de viscosité inférieure à 0,1 m².s⁻¹, un polymère cationique associé à un terpolymère acrylique spécifique, défini ci-après. En effet, il a été constaté que l'utilisation dudit terpolymère acrylique dans les compositions de la présente invention permettait d'améliorer la stabilité des shampooings à base de polyorganosiloxanes de viscosité inférieure à 0,1 m².s⁻¹ tout en conférant aux matières kératiniques et notamment aux cheveux des propriétés cosmétiques satisfaisantes particulièrement au niveau du démêlage, de la souplesse, de la malléabilité et du gonflant.

L'aspect général de la chevelure est également plus lisse au regard.

Il a été également constaté que les compositions de la présente invention présentaient une bonne tolérance cutanée.

L'invention a donc pour objet des compositions de lavage des matières kératiniques essentiellement caractérisées en ce qu'elles comprennent dans un milieu cosmétiquement acceptable:
i) au moins un agent tensio-actif détergent;
ii) au moins un polyorganosiloxane de viscosité inférieure à 0,1 m².s⁻¹;
iii) au moins un polymère cationique; et
iv) au moins un terpolymère acrylique constitué:
   - de 5 à 80% en poids, préférentiellement de 15 à 70% en poids et plus préférentiellement de 40 à 70% en poids, d'un monomère acrylate (a) choisi parmi un acrylate d'alkyle en C₁-C₆ et un méthacrylate d'alkyle en C₁-C₆;
   - de 5 à 80% en poids, préférentiellement de 10 à 70% en poids et plus préférentiellement de 20 à 60% en poids, d'un monomère (b) choisi parmi un composé vinylique héterocyclique contenant au moins un atome d'azote ou de soufre, un (méth)acrylamide, un (méth)acrylate de mono- ou di-(C₁-C₄)alkylamino(C₁-C₄)alkyle et un mono ou di-(C₁-C₄)alkylamino (C₁-C₄) alkyl (méth)acrylamide;
   - de 0,1 à 30% en poids, préférentiellement de 0,1 à 10% en poids d'un monomère (c) choisi parmi :
      un uréthane produit par réaction entre un isocyanate insaturé monoéthylénique et un agent tensioactif non ionique englobant un copolymère séquencé d'oxyde de 1,2-butylène et d'oxyde d'éthylène à extrémité alcoxy en C₁₋₄;
      un monomère tensioactif insaturé éthylénique copolymérisable obtenu par condensation d'un tensioactif non-ionique avec un acide carboxylique insaturé α,β-éthylénique ou son anhydride;
      un monomère tensioactif choisi parmi les produits de réaction de type urée d'un monoisocyanate insaturé monoéthylénique avec un tensioactif non-ionique présentant une fonction amine;
      un éther de (méth)allyle de formule CH₂=CR₁CH₂OAₘBₙAₚR₂ dans lequel R₁ désigne un atome d'hydrogène ou un groupe méthyle, A désigne un groupement propylèneoxy ou butylèneoxy, B désigne éthylèneoxy, n est égal à zéro ou désigne un nombre entier inférieur ou égal à 200 et préférentiellement inférieur ou égal à 100, m et p désignent zéro ou un nombre entier inférieur à n et R₂ est un groupe hydrophobe d'au moins 8 atomes de carbone et préférentiellement en C₈-C₃₀; et
      un monomère non-ionique de type uréthane produit par réaction d'un tensioactif non ionique monohydrique avec un isocyanate insaturé monoéthylénique;
      les pourcentages en poids de monomères étant basés sur le poids total des monomères constituant le terpolymère.

Dans la composition de lavage de l'invention, le terpolymère acrylique est présent à raison de 0,01 à 20 % en poids de matière active (M.A.), de préférence 0,1 à 10 % en poids par rapport au poids total de la composition.

Des monomères acrylates (a) préférés comprennent notamment les acrylates d'alkyle en C₂-C₆. L'acrylate d'éthyle est tout particulièrement préféré.

Comme exemples de monomères (b) préférés, il faut citer le méthacrylate de N,N-diméthylaminoéthyle (DMAEMA), l'acrylate de N,N-diéthylaminoéthyle, le méthacrylate de N,N-diéthylaminoéthyle, l'acrylate de N-t-butylaminoéthyle, le méthacrylate de N-t-butylaminoéthyle, le N,N-diméthylaminopropyl-acrylamide, le N,N-diméthylaminopropyle-méthacrylamide, le N,N-diéthylaminopropylacrylamide et le N,N-diéthylaminopropyl-méthacrylamide. Le méthacrylate de N,N-diméthylaminoéthyle est tout particulièrement préféré.

Les monomères (c) préférés sont les monomères tensio-actifs insaturés éthyléniques copolymérisables obtenus par condensation d'un tensioactif non-ionique avec un acide carboxylique insaturé α,β-éthylénique ou son anhydride, de préférence les acides mono ou dicarboxyliques en C₃-C₄ ou leurs anhydrides et plus particulièrement l'acide acrylique, l'acide méthacrylique, l'acide crotonique, l'acide maléique, l'anhydride maléique et tout particulièrement l'acide itaconique et l'anhydride itaconique.

Les monomères (c) particulièrement préférés correspondent aux monomères tensioactifs insaturés éthyléniques copolymérisables obtenus par condensation d'un tensioactif non-ionique avec l'acide itaconique. Parmi les tensioactifs non-ioniques, on peut citer notamment les alcools gras en C₁₀-C₃₀ alkoxylés avec 2 à 100, et de préférence de 5 à 50 moles d'oxyde d'alkylène, comme par exemple les éthers de polyéthylène glycol et d'alcools gras en C₁₀-C₃₀ et plus particulièrement les éthers de polyéthylène glycol et d'alcool cétylique, dénommés CETETH dans le dictionnaire CTFA, 7ème édition, 1997.

Des méthodes conventionnelles pour préparer ces terpolymères acryliques sont connues de l'homme du métier. De telles méthodes incluent la polymérisation en solution, la polymérisation par précipitation et la polymérisation en émulsion par exemple. Des terpolymères conformes à l'invention et leurs méthodes de préparation sont notamment décrits dans les demandes EP-A-0824914 et EP-A-0825200.

Parmi ces terpolymères, on préfère utiliser en particulier le polymère "STRUCTURE ® PLUS" vendu par la Société NATIONAL STARCH, qui est constitué d'acrylates, d'amino(méth)acrylates et d'itaconate d'alkyle en C₁₀-C₃₀ polyoxyéthyléné à 20 moles d'oxyde d'éthylène sous forme de dispersion aqueuse à 20% de M.A..

En plus de ces monomères, le terpolymère peut contenir d'autres monomères qui permettent de réticuler ledit terpolymère. Ces monomères sont utilisés dans des proportions assez faibles, jusqu'à 2% en poids par rapport au poids total des monomères utilisés pour préparer le terpolymère. De tels monomères de réticulation comprennent des monomères aromatiques portant plusieurs substituants vinyle, des monomères alicycliques portant plusieurs substituants vinyle, des esters bi-fonctionnels d'acide phtalique, des esters bi-fonctionnels d'acide méthacrylique, des esters multifonctionnels d'acide acrylique, le N-méthylène-bis-acrylamide et des monomères aliphatiques portant plusieurs substituants vinyle tels que des diènes, triènes et tétraènes. Des monomères de réticulation peuvent notamment être des divinyl-benzènes, des trivinyl-benzènes, le 1,2,4-trivinylcyclohexène, le 1,5-hexadiène, le 1,5,9-décatriène, le 1,9-décadiène, le 1,5-heptadiène, des di-allyl phthalates, de l'éthylène glycol diméthacrylate, des polyéthylène glycol diméthacrylates, des penta- et tétra-acrylates, des triallyl pentaérythritols, des octaallyl saccharoses, des cycloparaffines, des cyclooléfines et du N-méthylène-bis-acrylamide.

La viscosité est mesurée de préférence par viscométrie capillaire, par exemple à l'aide d'un viscomètre capillaire, notamment de type Ubbelohde à une température de 25°C, selon la norme ASTM-D445-97. On peut aussi utiliser la méthode dite de la chute de bille.

Dans le cadre de la présente invention, on entend par polyorganosiloxanes de viscosité inférieure à 0,1 m².s⁻¹ des polyorganosiloxanes modifiés ou non, à savoir des huiles de polyorganosiloxanes telles quelles ou sous forme de solutions dans des solvants organiques ou encore sous forme d'émulsions ou de microémulsions. Les silicones ont de préférence une viscosité comprise entre 0,001 et 0,08 et plus particulièrement entre 0,01 et 0,08 m².s⁻¹.

Parmi les polyorganosiloxanes pouvant être utilisés conformément à la présente invention, on peut citer à titre non limitatif :
I. Les silicones volatiles : celles-ci possèdent un point d'ébullition compris entre 60°C et 260°C. Elles sont choisies parmi les silicones cycliques contenant de 3 à 7 atomes de silicium et de préférence 4 à 5. Il s'agit, par exemple, de l'octaméthyl/cyclotétrasiloxane vendu sous le nom de "VOLATILE SILICONE 7207" par UNION CARBIDE ou "SILBIONE 70045 V2" par RHONE POULENC, le décaméthylcyclopentasiloxane vendu sous le nom de "VOLATILE SILICONE 7158" par UNION CARBIDE, "SILBIONE 70045 V5 par RHONE POULENC, ainsi que leurs mélanges. On cite également les cyclocopolymères du type diméthylsiloxane/méthylalkylsiloxane, tel que la "SILICONE VOLATILE FZ 3109" vendue parla Société UNION CARBIDE, qui est un cyclocopolymère diméthylsiloxane/méthyloctylsiloxane.
II. Les silicones non volatiles : elles sont constituées principalement par:
   (i) les polyalkylsiloxanes ; Parmi les polyalkylsiloxanes, on peut citer principalement les polydiméthylsiloxanes linéaires à groupements terminaux triméthylsilyle, comme par exemple, et à titre non limitatif, les huiles "SILBIONE" de la série 70047 commercialisées par RHODIA CHIMIE; les polydiméthylsiloxanes DC 200 de viscosité 0,03 ou 0,06 m².s⁻¹ de DOW CORNING;
   (ii) les polyarylsiloxanes ;
   (iii) les polyalkylarylsiloxanes ; on peut citer les polyméthylphénylsiloxanes, les polydiméthylméthylphénylsiloxanes, les polydiméthyldiphénylsiloxanes linéaires et ramifiés, tels que par exemple l'huile "RHODORSIL 763" de RHODIA CHIMIE;
   (iv) les polyorganosiloxanes organomodifiés ; c'est à dire des silicones telles que définies précédemment, comportant dans leur structure générale, un ou plusieurs groupements organofonctionnels directement fixés sur la chaîne siloxanique ou fixés par l'intermédiaire d'un radical hydrocarboné; on cite, par exemple, les silicones comportant :
      a) des groupements polyéthylèneoxy et/ou polypropylèneoxy, comportant éventuellement des groupes alkyle, tels que le produit dénommé diméthicone copolyol vendu par la Société DOW CORNING sous la dénomination "DC 1248", et l'alkyl (C12) méthicone copolyol vendu par la Société DOW CORNING sous la dénomination "Q2 5200";
      b) des groupements (per)fluorés comme les groupements trifluoroalkyle, telles que, par exemple, celles vendues par la Société GENERAL ELECTRIC sous les dénominations "FF.150 Fluorosilicone Fluid";
      c) des groupements hydroxyacylamino, telles que celles décrites dans la demande de brevet européen EP-A-0 342 834 et en particulier la silicone vendue par la Société DOW CORNING sous la dénomination "Q2-8413" ;
      d) des groupements thiols comme dans les silicones "X 2-8360" de DOW CORNING ou les "GP 72A" et "GP 71" de GENESEE ;
      e) des groupements aminés substitués ou non, comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ou aminoalkyl(C₁-C₄)aminoalkyl(C₁-C₄). On utilise plus particulièrement les silicones dénommées amodiméthicone et triméthylsilylamodiméthicone selon la dénomination CTFA (1997);
      f) des groupements carboxylates, comme les produits décrits dans le brevet européen EP 186 507 de CHISSO CORPORATION;
      g) des groupements hydroxylés, comme les polyorganosiloxanes à fonction hydroxyalkyle, décrits dans la demande de brevet FR-A-2 589 476 ;
      h) des groupements alcoxylés comportant au moins 12 atomes de carbone comme le produit "SILICONE COPOLYMER F 755" de SWS SILICONES;
      i) des groupements acyloxyalkyle comportant au moins 12 atomes de carbone, comme par exemple les polyorganosiloxanes décrits dans la demande de brevet FR-A-2 641 185;
      j) des groupements ammonium quaternaire, comme dans le produit "ABIL K 3270" de la Société GOLDSCHMIDT ;
      k) des groupements amphotères ou bétaïniques, tels que dans le produit vendu par la Société GOLDSCHMIDT sous la dénomination "ABIL B 9950" ;
      l) des groupements bisulfite, tels que dans les produits vendus par la Société GOLDSCHMIDT sous les dénominations "ABIL S 201 " et "ABIL S 255";
   (v) les copolymères blocs ayant un bloc linéaire polysiloxane-polyalkylène comme unité répétitive ; la préparation de tels copolymères blocs mis en oeuvre dans le cadre de la présente invention est décrite dans la demande européenne EP 0 492 657 A1.
   (vi) les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ; notamment ceux choisis plus préférentiellement parmi ceux décrits dans les brevets US 4.963.935, US 4.728.571 et US 4.972.037 et les demandes de brevet EP-A-0 412 704, EP-A-0 412 707, EP-A-0 640 105 et WO 95/00578;
   (vii) les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ; des exemples de tels polymères, ainsi que leur mode particulier de préparation, sont notamment décrits dans les demandes de brevets EP-A-0 582 152, WO 93/23009 et WO 95/03776;
   (viii) ou leurs mélanges.

Les polyorganosiloxanes préférés pour être utilisés selon l'invention sont les polyorganopolysiloxanes non volatils et en particulier les polydiméthylsiloxanes, aminés ou non.

Les polyorganosiloxanes de viscosité inférieure à 0,1 m².s⁻¹ peuvent être présents dans des proportions comprises entre 0,01% et 20% en poids par rapport au poids total de la composition et préférentiellement dans des proportions comprises entre 0,1% et 10% en poids par rapport au poids total de la composition.

Comme indiqué précédemment, les compositions selon l'invention contiennent au moins un agent tensio-actif détergent, notamment choisi parmi les tensio-actifs anioniques, amphotères, non-ioniques et cationiques ayant des propriétés détergentes, et leurs mélanges.

Parmi les agents tensio-actifs anioniques, on peut citer les sels alcalins, les sels d'ammonium, les sels d'amines, les sels d'aminoalcools, les sels de magnésium des composés suivants : les alkylsulfates, alkyléthersulfates, alkylamidoéthersulfates, alkylarylpolyéthersulfates, monoglycérides sulfates; les alkylsulfonates, alkylamides sulfonates, alkylarylsulfonates, oléfines sulfonates, paraffines sulfonates; les alkylsulfosuccinates, les alkyléthersulfosuccinates, les alkylamides sulfosuccinates; les alkylsulfosuccinamates; les alkylsulfoacétates; les alkylphosphates, alkyléther phosphates; les acylsarcosinates, les acyliséthionates et les N-acyltaurates.

Le radical alkyle ou acyle de ces différents composés est généralement constitué par une chaîne carbonée comportant de 8 à 30 atomes de carbone.

Parmi les agents tensio-actifs anioniques, on peut également citer les sels d'acides gras tels que les sels des acides oléique, ricinoléique, palmitique, stéarique; les acides d'huile de coprah ou d'huile de coprah hydrogénée; les acyl lactylates, dont le radical acyle comporte de 8 à 30 atomes de carbone.

On peut également utiliser des agents tensio-actifs considérés comme faiblement anioniques tels que les acides alkyl ou alkylaryl éther carboxyliques polyoxyalkylénés ou leurs sels, les acides alkylamido éther carboxyliques polyoxyalkylénés ou leurs sels, les acides d'alkyl D-galactoside uroniques ou leurs sels.

Les agents tensio-actifs non-ioniques sont plus particulièrement choisis parmi les alcools ou les alkylphénols ou les acides gras polyéthoxylés, polypropoxylés ou polyglycérolés, à chaîne grasse comportant 8 à 30 atomes de carbone, le nombre de groupements oxyde d'éthylène ou oxyde de propylène étant compris entre 2 et 50 et le nombre de groupements glycérol étant compris entre 2 et 30.

On peut également citer les copolymères d'oxydes d'éthylène et de propylène; les condensats d'oxydes d'éthylène et de propylène sur des alcools gras; les amides gras polyéthoxylés ayant de préférence 2 à 30 moles d'oxyde d'éthylène; les amides gras polyglycérolés comportant de préférence 1 à 5 groupements glycérol et en particulier 1,5 à 4; les amines grasses polyéthoxylées ayant de préférence 2 à 30 moles d'oxyde d'éthylène; les esters d'acides gras du sorbitan oxyéthylénés avec 2 à 30 moles d'oxyde d'éthylène; les esters d'acide gras de sucrose, les esters d'acides gras du polyéthylèneglycol, les alkylpolyglycosides, les dérivés carbamates ou amides de N-alkyl glucamines, les aldobionamides, les oxydes d'amines tels que les oxydes d'alkylamines ou de N-acylamidopropyl-morpholine.

Les agents tensio-actifs amphotères préférés sont les dérivés d'amines secondaires ou tertiaires aliphatiques, dans lesquels le radical aliphatique est une chaîne linéaire ou ramifiée comportant 8 à 22 atomes de carbone et qui contient au moins un groupe anionique hydrosolubilisant, carboxylate, sulfonate, sulfate, phosphate ou phosphonate; les alkyl(C₈-C₂₀)bétaïnes, les sulfobétaïnes, les alkyl(C₈-C₂₀)amidoalkyl(C₁-C₆) bétaïnes ou les alkyl(C₈-C₂₀)amidoalkyl(C₁-C₆)sulfobétaïnes.

Parmi les dérivés d'amines, on peut citer les produits vendus sous la dénomination MIRANOL, tels que décrits dans les brevets US-A-2 528 378 et 2 781 354 et classés dans le dictionnaire CTFA, 7ème édition, 1997, sous la dénomination Disodium Cocoamphodiacétate, Disodium Lauroamphodiacétate, Disodium Capryloamphodiacétate, Disodium Caproamphodiacétate, Disodium Cocoamphodipropionate, Disodium Lauroamphodipropionate, Disodium Caproamphodipropionate, Disodium Capryloamphodipropionate, Lauroamphodipropionate acide, Cocoamphodipropionate acide.

Les agents tensio-actifs cationiques sont notamment choisis parmi les sels d'amines grasses primaires, secondaires ou tertiaires, éventuellement polyoxyalkylénées; les sels d'ammonium quaternaire; les dérivés d'imidazoline; ou les oxydes d'amines à caractère cationique.

Les sels d'ammonium quaternaire préférés sont les halogénures (par ex. chlorures) de tétraalkylammonium comme par exemple les chlorures de dialkyldiméthylammonium ou d'alkyltriméthylammonium, dans lesquels le radical alkyl comporte de 12 à 22 atomes de carbone, en particulier les chlorures de béhényltriméthylammonium, de distéaryldiméthylammonium, de cétyltriméthylammonium, de benzyl diméthyl stéaryl ammonium ou encore le chlorure de stéaramidopropyldiméthyl (myristyl acétate) ammonium commercialisé sous la dénomination «CEPHARYL 70» par la société VAN DYK.

On peut également utiliser les sels (chlorures ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol.

Les agents tensio-actifs sont utilisés dans les compositions conformes à l'invention dans des proportions suffisantes pour conférer un caractère détergent à la composition, généralement à raison d'au moins 4% en poids, de préférence entre 5 et 50% en poids par rapport au poids total de la composition et en particulier entre 8 et 35%.

Les compositions de l'invention contiennent en outre au moins un polymère cationique choisi parmi tous ceux déjà connus en soi, notamment ceux décrits dans la demande de brevet EP-A-0 337 354 et dans les demandes de brevets français FR-A-2 270 846, 2 383 660, 2 598 611, 2 470 596 et 2 519 863.

Les polymères cationiques utilisés ont généralement une masse moléculaire comprise entre 500 et 5.10⁶ environ, et de préférence comprise entre 10³ et 3.10⁶ environ .

Parmi les polymères cationiques, on peut citer les protéines (ou hydrolysats de protéines) quaternisées et les polymères du type polyamine, polyaminoamide et polyammonium quaternaire. Ce sont des produits connus.

Les protéines ou hydrolysats de protéines quaternisés sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment :
- les hydrolysats de collagène portant des groupements triéthylammonium tels que les produits dénommés dans le dictionnaire CTFA "TRIETHONIUM HYDROLYZED COLLAGEN ETHOSULFATE" ;
- les hydrolysats de collagène portant des groupements chlorure de triméthylammonium et de triméthylstéarylammonium, dénommés dans le dictionnaire CTFA "STEARTRIMONIUM HYDROLYZED COLLAGEN" ;
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres le "CROQUAT L", le "CROQUAT M", le "CROQUAT S" et le "CROTEIN Q" vendus par la Société CRODA.

D'autre protéines ou hydrolysats quaternisés sont par exemple ceux vendus par la Société INOLEX, sous la dénomination "LEXEIN QX 3000".

On peut encore citer les protéines végétales quaternisées telles que les protéines de blé, de maïs ou de soja: comme protéines de blé quaternisées, on peut citer celles appelées dans le dictionnaire CTFA "COCODIMONIUM HYDROLYSED WHEAT PROTEIN", "LAURIDIMONIUM HYDROLYSED WHEAT PROTEIN", ou encore "STEARDIMONIUM HYDROLYSED WHEATPROTEIN".

Les polymères du type polyamine, polyaminoamide, polyammonium quaternaire, utilisables conformément à la présente invention, pouvant être notamment mentionnés, sont ceux décrits dans les brevets français n°2 505 348 ou 2 542 997. Parmi ces polymères, on peut citer:
(1) Les copolymères vinylpyrrolidone-acrylate ou - méthacrylate de dialkylaminoalkyle quaternisés ou non, tels que les polymères décrits en détail dans les brevets français 2 077 143 et 2 393 573.
(2) Les dérivés d'éthers de cellulose comportant des groupements ammonium quaternaires décrits dans le brevet français 1 492 597.
(3) Les dérivés de cellulose cationiques tels que les copolymères de cellulose ou les dérivés de cellulose greffés avec un monomère hydrosoluble d'ammonium quaternaire, et décrits notamment dans le brevet US 4131 576, tels que les hydroxyalkyl celluloses, comme les hydroxyméthyl-, hydroxyéthyl- ou hydroxypropyl celluloses greffées notamment avec un sel de méthacryloyléthyl triméthyl ammonium, de méthacrylamidopropyl triméthyl ammonium ou de diméthyldiallylammonium.
(4) Les polysaccharides et notamment gommes de guar cationiques décrits plus particulièrement dans les brevets US 3 589 578 et 4 031 307.
(5) Les polymères constitués de motifs pipérazinyle et de radicaux divalents alkylène ou hydroxyalkylène à chaînes droites ou ramifiées, éventuellement interrompues par des atomes d'oxygène, de soufré, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé, l'agent réticulant étant utilisé dans des proportions allant de 0,025 à 0,35 mole par groupement amine du polyaminoamide; ces polyaminoamides peuvent être alcoylés ou s'ils comportent une ou plusieurs fonctions amines tertiaires, quaternisés. De tels polymères sont notamment décrits dans les brevets français 2.252.840 et 2.368.508.
(7) Les dérivés de polyaminoamides résultant de la condensation de polyalcoylène polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone, le rapport molaire entre la polyalkylène polyamine et l'acide dicarboxylique étant compris entre 0,8 : 1 et 1,4 : 1, le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
(9) Les cyclopolymères de méthyl diallyl amine ou de diméthyl diallyl ammonium, notamment ceux décrits dans le brevet français 2.080.759 et dans son certificat d'addition 2.190.406.
(10) Les polymères de diammonium quaternaire décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
(11) Les polymères de polyammonium quaternaire notamment décrits dans la demande de brevet EP-A-122 324.
(12) Les homopolymères ou copolymères dérivés des acides acrylique ou méthacrylique et comportant des motifs CH₂-CHRₐ-CO-O-A₁-NRₑR_{f} , CH₂-CHRₐ-CO-O-A₁-N⁺R_{b}R_{c}R_{d}, X⁻ et/ou CH₂-CHRₐ-CO-NH-A₁-N⁺R_{b}R_{c}R_{d}, X⁻ dans lesquels les groupements Rₐ désignent indépendamment H ou CH₃, les groupements A₁ désignent indépendamment un groupe alcoyle linéaire ou ramifié de 1 à 6 atomes de carbone ou un groupe hydroxyalcoyle de 1 à 4 atomes de carbone, les groupements R_{b}, R_{c}, R_{d}, identiques ou différents, désignent indépendamment un groupe alcoyle de 1 à 18 atomes de carbone ou un radical benzyle, les groupements Rₑ et R_{f} représentent un atome d'hydrogène ou un groupement alcoyle de 1 à 6 atomes de carbone, X⁻ désigne un anion, par exemple méthosulfate ou halogénure, tel que chlorure ou bromure,
(13) Les polymères quaternaires de vinylpyrrolidone et de vinylimidazole tels que par exemple les produits commercialisés sous les dénominations "LUVIQUAT FC 905", "LUVIQUAT FC 550" et "LUVIQUAT FC 370" par la société BASF.
(14) Les polyamines comme le "POLYQUART H" vendu par HENKEL, référencé sous le nom de « POLYETHYLENEGLYCOL TALLOW POLYAMINE » dans le dictionnaire CTFA.
(15) Les polymères réticulés de sel de méthacryloyloxyéthyl triméthylammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléhnique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère les dérivés d'éther de cellulose comportant des groupements ammonium quaternaires, les polysaccharides et notamment gomme de guar cationiques et les cyclopolymères de méthyl diallyl amine ou de diméthyl diallyl ammonium.

Les polymères cationiques sont utilisés dans les compositions de l'invention dans des proportions comprises entre 0,001 et 20% en poids et de préférence entre 0,05 et 5% en poids par rapport au poids total de la composition.

Les compositions, selon l'invention, présentent un pH généralement compris entre 3 et 12, et plus particulièrement entre 4 et 8.

Le milieu cosmétiquement acceptable des compositions est constitué soit par de l'eau, soit par un ou plusieurs solvants, soit par un mélange d'eau et d'au moins un solvant choisi parmi les alcools inférieurs, les alkylèneglycols et les éthers de polyols.

Les performances cosmétiques des compositions selon l'invention peuvent être améliorées par ajout de polyorganosiloxanes différents des polyalkylsiloxanes décrits ci-dessus de viscosité supérieure à 0,1 m²/s et notamment des résines ou gommes de silicone.

Les compositions selon l'invention peuvent également contenir en outre au moins un adjuvant choisi parmi les adjuvants habituellement utilisés en cosmétique, tel que les parfums, les conservateurs, les séquestrants, les humectants, les sucres, les huiles végétales, minérales, animales ou de synthèse, les polymères amphotères, le menthol, les dérivés de nicotinate, les agents antichute des cheveux, les agents antipelliculaires, les stabilisateurs de mousse, les agents propulseurs, les filtres, les colorants, les céramides, les vitamines ou provitamines, telles que le panthénol, la vitamine E, les agents acidifiants ou alcalinisants ou d'autres adjuvants cosmétiques bien connus.

Dans une forme de réalisation préférée de l'invention, les compositions selon l'invention sont utilisées comme shampooings pour le lavage des cheveux.

Le procédé de lavage des matières kératiniques consiste à appliquer une composition telle que définie ci-dessus sur des matières kératiniques humides ou sèches dans des quantités efficaces pour les laver, cette application étant suivie d'un rinçage après un temps de pose facultatif.

L'exemple qui suit est destiné à illustrer l'invention.

### EXEMPLE DE SHAMPOOING

| | |
|---|---|
| Propylène glycol | 0,1 g |
| N-cocoyl amidoéthyl, N-éthoxycarboxyméthyl glycinate de sodium à 38% de M.A. vendu sous la dénomination " MIRANOL C2M Conc" par la société RHODIA | 8 g |
| Chlorure d'hydroxypropyl guar triméthyl ammonium vendu sous la dénomination "JAGUAR C13S" par la société MEYHALL | 0,2 g |
| Mélange 1-(hexadécyloxy)-2-octadécanol/ alcool cétylique | 2,5 g |
| Monoisopropanolamide d'acides de coprah | 0,5 g |
| Lauryl éther sulfate de sodium (2,2 OE) à 70% M.A. | 22 g |
| Polydiméthylsiloxane de viscosité 0,06 m².s⁻¹ vendu sous la dénomination "DC 200 FLUID - 60.000 cSt" par la société DOW CORNING | 2,7 g |
| Terpolymère d'acrylates, d'amino(méth)acrylates et d'itaconate d'alkyle en C₁₀-C₃₀ polyoxyéthyléné à 20 moles d'oxyde d'éthylène en dispersion aqueuse à 20% M.A. vendu sous la dénomination "STRUCTURE® PLUS" par la Société National Starch | 1 g |
| Parfum, conservateurs qs | |
| Eau déminéralisée stérilisée qsp | 100 g |

On ajuste le pH à 7,5 par de l'acide citrique ou de l'hydroxyde de sodium.

Après application de ce shampooing, on constate sur cheveux séchés: de la souplesse, de la malléabilité et du gonflant.
Les cheveux sont aussi faciles à démêler.

## Revendications

1. Composition de lavage des matières kératiniques comprenant dans un milieu cosmétiquement acceptable, au moins un agent tensio-actif détergent, au moins un polymère cationique et au moins une huile de polyorganosiloxane de viscosité inférieure ou égale à 0,1 m².s⁻¹, **caractérisée par le fait qu'**elle comprend en outre au moins un terpolymère acrylique constitué:
- de 5 à 80% en poids, préférentiellement de 15 à 70% en poids et plus préférentiellement de 40 à 70% en poids, d'un monomère acrylate (a) choisi parmi un acrylate d'alkyle en C₁-C₆ et un méthacrylate d'alkyle en C₁-C₆;
- de 5 à 80% en poids, préférentiellement de 10 à 70% en poids et plus préférentiellement de 20 à 60% en poids, d'un monomère (b) choisi parmi un composé vinylique héterocyclique contenant au moins un atome d'azote ou de soufre, un (méth)acrylamide, un (méth)acrylate de mono- ou di-(C₁-C₄)alkylamino(C₁-C₄)alkyle et un mono ou di (C₁-C₄)alkylamino (C₁-C₄) alkyl (méth)acrylamide;
- de 0,1 à 30% en poids, préférentiellement de 0,1 à 10% en poids d'un monomère (c) choisi parmi :
un uréthane produit par réaction entre un isocyanate insaturé monoéthylénique et un agent tensioactif non ionique englobant un copolymère séquencé d'oxyde de 1,2-butylène et d'oxyde d'éthylène à extrémité alcoxy en C₁₋₄ ;
un monomère tensioactif insaturé éthylénique copolymérisable obtenu par condensation d'un tensioactif non-ionique avec un acide carboxylique insaturé α,β-éthylénique ou son anhydride;
un monomère tensioactif choisi parmi les produits de réaction de type urée d'un monoisocyanate insaturé monoéthylénique avec un tensioactif non ionique présentant une fonctionalité amine;
un éther de (méth)allyle de formule CH₂=CR₁CH₂OAₘBₙAₚR₂ dans lequel R₁ désigne un atome d'hydrogène ou un groupe méthyle, A désigne un groupement propylèneoxy ou butylèneoxy, B désigne éthylèneoxy, n est égal à zéro ou désigne un nombre entier inférieur ou égal à 200, m et p désignent zéro ou un nombre entier inférieur à n et R₂ est un groupe hydrophobe d'au moins 8 atomes de carbone; et
un monomère non-ionique de type uréthane produit par réaction d'un tensioactif non ionique monohydrique avec un isocyanate insaturé monoéthylénique;
les pourcentages en poids de monomères étant basés sur le poids total des monomères constituant le terpolymère.

2. Composition selon la revendication 1, **caractérisée en ce que** le terpolymère est présent à raison de 0,01 à 20% en poids de matière active, de préférence 0,1 à 10 % en poids par rapport au poids total de la composition.

3. Composition selon la revendication 1 ou 2, **caractérisée en ce que** le monomère (a) est choisi parmi les acrylates d'alkyle en C₂-C₆, et est préférentiellement l'acrylate d'éthyle.

4. Composition selon l'une quelconque des revendications 1 à 3, **caractérisée en ce que** le monomère (b) est choisi parmi le méthacrylate de N,N-diméthylaminoéthyle, l'acrylate de N,N-diéthylaminoéthyle, le méthacrylate de N,N-diéthylaminoéthyle, l'acrylate de N-t-butylaminoéthyle, le méthacrylate de N-t-butylaminoéthyle, le N,N-diméthylaminopropyl-acrylamide, le N,N-diméthylaminopropylméthacrylamide, le N,N-diéthylaminopropyl-acrylamide et le N,N-diéthylaminopropyl-méthacrylamide, et est préférentiellement le méthacrylate de N,N-diméthylaminoéthyle.

5. Composition selon l'une quelconque des revendications 1 à 4, **caractérisée en ce que** le monomère (c) est un monomère tensioactif insaturé éthylénique copolymérisable obtenu par condensation d'un tensioactif non-ionique avec l'acide itaconique.

6. Composition selon l'une quelconque des revendications 1 à 5, **caractérisée en ce que** le terpolymère acrylique est constitué d'acrylates, d'amino(méth)acrylates et d'itaconate d'alkyle en C₁₀-C₃₀ polyoxyéthyléné à 20 moles d'oxyde d'éthylène.

7. Composition selon l'une quelconque des revendications 1 à 6, **caractérisée en ce que** le terpolymère acrylique contient en outre un monomère de réticulation.

8. Composition selon l'une quelconque des revendications 1 à 7, **caractérisée par le fait que** le polyorganosiloxane de viscosité inférieure à 0,1 m².s⁻¹ est choisi parmi les silicones volatiles et les silicones non-volatiles et notamment:
(i) les polyalkylsiloxanes;
(ii) les polyarylsiloxanes;
(iii) les polyalkylarylsiloxanes;
(iv) les polyorganosiloxanes organomodifiés ;
(v) les copolymères blocs ayant un bloc linéaire polysiloxanepolyalkylène comme unité répétitive ;
(vi) les polymères siliconés greffés, à squelette organique non siliconé;
(vii) les polymères siliconés greffés, à squelette polysiloxanique greffé par des monomères organiques non siliconés;
(viii) et leurs mélanges.

9. Composition selon l'une quelconque des revendications 1 à 8, **caractérisée en ce que** le polyorganosiloxane de viscosité inférieure à 0,1 m².s⁻¹ est choisi parmi les polydiméthylsiloxanes.

10. Composition selon l'une quelconque des revendications 1 à 9, **caractérisée par le fait que** le polyorganosiloxane de viscosité inférieure à 0,1 m².s⁻¹ est présent à raison de 0,01 à 20% en poids, de préférence de 0,1 à 10% en poids, par rapport au poids total de la composition.

11. Composition selon l'une quelconque des revendications 1 à 10, **caractérisée en ce que** l'agent tensio-actif détergent est choisi parmi les agents tensio-actifs anioniques, amphotères, non-ioniques, cationiques et leurs mélanges.

12. Composition selon la revendication 11, **caractérisée par le fait que** les agents tensio-actifs anioniques sont choisis parmi les sels alcalins, les sels de magnésium, les sels d'ammonium, les sels d'amines ou les sels d'aminoalcools des composés suivants : les alkylsulfates, alkyléthersulfates, alkylamidoéthersulfates, alkylarylpolyéthersulfates, monoglycérides sulfates; les alkylsulfonates, alkylamides sulfonates, alkylarylsulfonates, oléfines sulfonates, paraffines sulfonates; les alkylsulfosuccinates, les alkyléthersulfosuccinates, les alkylamides sulfosuccinates; les alkylsulfosuccinamates; les alkylsulfoacétates; les alkylphosphates, alkyléther phosphates; les acylsarcosinates, les acyliséthionates, N-acyltaurates; le radical alkyle ou acyle de ces différents composés étant constitué par une chaîne carbonée comportant de 8 à 30 atomes de carbone; les sels d'acides gras des acides oléique, ricinoléique, palmitique, stéarique; les acides d'huile de coprah ou d'huile de coprah hydrogénée; les acyl lactylates, dont le radical acyle comporte de 8 à 30 atomes de carbone; les acides d'alkyl D-galactoside uroniques et leurs' sels, les acides alkyl ou alkylaryl éther carboxyliques polyoxyalkylénés ou leurs sels, les acides alkylamido éther carboxyliques polyoxyalkylénés ou leurs sels.

13. Composition selon la revendication 11, **caractérisée par le fait que** les agents tensio-actifs non-ioniques sont choisis parmi les alcools ou les alkylphénols ou les acides gras polyéthoxylés, polyoxypropylénés ou polyglycérolés, à chaîne grasse comportant 8 à 30 atomes de carbone, le nombre de groupements oxyde d'éthylène, oxyde de propylène étant compris entre 2 et 50 et le nombre de groupements glycérol étant compris entre 2 et 30; les copolymères d'oxyde d'éthylène et de propylène; les condensats d'oxyde d'éthylène et de propylène sur des alcools gras; les amides gras polyéthoxylés; les amides gras polyglycérolés; les amines grasses polyéthoxylées; les esters d'acides gras du sorbitan oxyéthylénés; les esters d'acides gras de sucrose ou du polyéthylèneglycol; les alkylpolyglycosides; les dérivés amides ou carbamates de N-alkylglucamines, les aldobionamides et les oxydes d'amines.

14. Composition selon la revendication 11, **caractérisée par le fait que** les agents tensio-actifs amphotères sont choisis parmi les dérivés d'amines secondaires ou tertiaires aliphatiques, dans lesquels le radical aliphatique est une chaîne linéaire ou ramifiée comportant de 8 à 22 atomes de carbone et qui contient au moins un groupe anionique hydrosolubilisant, carboxylate, sulfonate, sulfate, phosphate, phosphonate; les alkyl(C₈-C₂₀)bétaïnes, les sulfobétaïnes, les alkyl(C₈-C₂₀)amidoalkyl(C₁-C₆) bétaïnes ou les alkyl(C₈-C₂₀)amidoalkyl(C₁-C₆)sulfobétaïnes.

15. Composition selon la revendication 11, **caractérisée par le fait que** les agents tensio-actifs cationiques sont choisis parmi les sels d'amines grasses primaires, secondaires ou tertiaires, éventuellement polyoxyalkylénées; les sels d'ammonium quaternaire; les dérivés d'imidazoline; et les oxydes d'amines à caractère cationique.

16. Composition selon l'une quelconque des revendications 1 à 15, **caractérisée en ce que** l'agent tensio-actif détergent est présent à raison d'au moins 4% en poids, de préférence de 5 à 50% en poids, et encore plus préférentiellement de 8 à 35% en poids par rapport au poids total de la composition.

17. Composition selon l'une quelconque des revendications 1 à 16, **caractérisée par le fait que** le polymère cationique est choisi parmi :
- les protéines ou hydrolysats de protéines, en particulier les hydrolysats de collagène portant des groupements triéthylammonium, les hydrolysats de collagène portant des groupements chlorure de triméthylammonium et de triméthylstéarylammonium, les hydrolysats de protéines portant sur la chaîne polypeptidique des groupements ammonium quaternaire comportant au moins un radical alkyle ayant de 1 à 18 atomes de carbone, les protéines végétales quaternisées telles que les protéines de blé, de maïs ou de soja;
- les polymères du type polyamine, polyaminoamide, polyammonium quaternaire, en particulier
i) les copolymères vinylpyrrolidone-acrylate ou méthacrylate de dialkylaminoalkyle quaternisés ou non,
ii) les dérivés d'éthers de cellulose comportant des groupements ammonium quaternaires,
iii) les dérivés de cellulose cationiques tels que les copolymères de cellulose ou les dérivés de cellulose greffés avec un monomère hydrosoluble d'ammonium quaternaire,
iv) les polysaccharides et notamment gommes de guar cationiques,
v) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères,
vi) les polyaminoamides solubles dans l'eau préparés en particulier par polycondensation d'un composé acide avec une polyamine,
vii) les dérivés de polyaminoamides résultant de la condensation de polyalcoylène polyamines avec des acides polycarboxyliques suivie d'une alcoylation par des agents bifonctionnels,
viii) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone,
ix) les cyclopolymères de méthyl diallyl amine ou de diméthyl diallyl ammonium,
x) les polymères de diammonium quaternaire,
xi) les polymères de polyammonium quaternaire,
xii) les homopolymères ou copolymères dérivés des acides acrylique ou méthacrylique et comportant des motifs CH₂-CHRₐ-CO-O-A₁-NRₑR_{f} , CH₂-CHRₐ-CO-O-A₁-N⁺R_{b}R_{c}R_{d}. X⁻ et/ou CH₂-CHRₐ-CO-NH-A₁-N⁺R_{b}R_{c}R_{d}, X⁻
dans lesquels les groupements Rₐ désignent indépendamment H ou CH₃, les groupements A₁ désignent indépendamment un groupe alcoyle linéaire ou ramifié de 1 à 6 atomes de carbone ou un groupe hydroxyalcoyle de 1 à 4 atomes de carbone, les groupements R_{b}, R_{c}, R_{d}, identiques ou différents, désignent indépendamment un groupe alcoyle de 1 à 18 atomes de carbone ou un radical benzyle, les groupements Rₑ et R_{f} représentent un atome d'hydrogène ou un groupement alcoyle de 1 à 6 atomes de carbone, X- désigne un anion, par exemple méthosulfate ou halogénure, tel que chlorure ou bromure,
xiii) les polymères quaternaires de vinylpyrrolidone et de vinylimidazole,
xiv) les polyamines référencé sous le nom de « POLYETHYLENEGLYCOL TALLOW POLYAMINE » dans le dictionnaire CTFA,
xv) les polymères réticulés de sel de méthacryloyloxyéthyl triméthylammonium,
- les polyalkylèneimines, les polymères contenant des motifs vinylpyridine ou vinylpyridinium, les condensats de polyamines et d'épichlorhydrine, les polyuréylènes quaternaires et les dérivés de la chitine.

18. Composition selon l'une quelconque des revendications 1 à 17, **caractérisée en ce que** le polymère cationique est choisi parmi les dérivés d'éther de cellulose comportant des groupements ammonium quaternaires, les gommes de guar cationiques et les cyclopolymères de méthyl diallyl amine ou de diméthyl diallyl ammonium.

19. Composition selon l'une quelconque des revendications 1 à 18, **caractérisée par le fait qu'**elle contient un polymère cationique dans des proportions comprises entre 0,001 et 20% en poids et de préférence entre 0,05 et 5% en poids par rapport au poids total de la composition.

20. Composition selon l'une quelconque des revendications 1 à 19, **caractérisée en ce qu'**elle présente un pH compris entre 3 et 12, et plus particulièrement entre 4 et 8.

21. Composition selon l'une quelconque des revendications 1 à 20, **caractérisée par le fait que** le milieu cosmétiquement acceptable est constitué par de l'eau ou par un ou plusieurs solvants ou par un mélange d'eau et d'au moins un solvant choisi parmi les alcools inférieurs, les alkylèneglycols et les éthers de polyol.

22. Composition selon l'une quelconque des revendications 1 à 21, **caractérisée par le fait qu'**elle contient en outre au moins une résine ou gomme de silicone.

23. Composition selon l'une quelconque des revendications 1 à 22, **caractérisée par le fait qu'**elle contient en outre au moins un adjuvant cosmétiquement acceptable choisi parmi les parfums, les conservateurs, les séquestrants, les humectants, les sucres, les huiles végétales, minérales, animales ou de synthèse, les polymères amphotères, le menthol, les dérivés de nicotinates, les agents antichute des cheveux, les agents antipelliculaires, les stabilisateurs de mousse, les agents propulseurs, les colorants, les filtres, les céramides, les vitamines ou provitamines et les agents acidifiants ou alcalinisants.

24. Utilisation comme shampooing de la composition telle que définie dans l'une quelconque des revendications 1 à 23.

25. Procédé de lavage des matières kératiniques, **caractérisé par le fait que** l'on applique sur les matières kératiniques humides ou sèches au moins une composition telle que définie dans l'une quelconque des revendications 1 à 23, et après un temps de pose facultatif, on les rince à l'eau.

## Claims

1. Composition for washing keratin materials, comprising, in a cosmetically acceptable medium, at least one detergent surfactant, at least one cationic polymer and at least one polyorganosiloxane oil with a viscosity of less than or equal to 0.1 m².s⁻¹, **characterized in that** it also comprises at least one acrylic terpolymer consisting of:
- from 5% to 80% by weight, preferably from 15% to 70% by weight and more preferably from 40% to 70% by weight, of an acrylate monomer (a) chosen from a C₁-C₆ alkyl acrylate and a C₁-C₆ alkyl methacrylate;
- from 5% to 80% by weight, preferably from 10% to 70% by weight and more preferably from 20% to 60% by weight, of a monomer (b) chosen from a heterocyclic vinyl compound containing at least one nitrogen or sulphur atom, a (meth)acrylamide, a mono- or di (C₁-C₄) alkylamino (C₁-C₄) alkyl (meth) acrylate and a mono- or di(C₁-C₄)alkylamino(C₁-C₄) alkyl (meth) acrylamide;
- from 0.1% to 30% by weight, preferably from 0.1% to 10% by weight, of a monomer (c) chosen from:
a urethane produced by reaction between a monoethylenic unsaturated isocyanate and a nonionic surfactant encompassing a block copolymer of 1,2-butylene oxide and of ethylene oxide containing a C₁₋₄ alkoxy end;
a copolymerizable ethylenic unsaturated surfactant monomer obtained by condensing a nonionic surfactant with an α,β-ethylenic unsaturated carboxylic acid or its anhydride;
a surfactant monomer chosen from reaction products such as urea of a monoethylenic unsaturated monoisocyanate with a nonionic surfactant containing an amine function;
a (meth) allyl ether of formula CH₂=CR₁CH₂OAₘBₙAₚR₂ in which R₁ denotes a hydrogen atom or a methyl group, A denotes a propylenoxy or butylenoxy group, B denotes ethylenoxy, n is equal to zero or denotes an integer less than or equal to 200, m and p denote zero or an integer less than n and R₂ is a hydrophobic group of at least 8 carbon atoms and preferably of C₈-C₃₀; and
a nonionic monomer such as urethane produced by reaction of a monohydric nonionic surfactant with a monoethylenic unsaturated isocyanate;
the weight percentages of monomers being based on the total weight of the monomers constituting the terpolymer.

2. Composition according to Claim 1, **characterized in that** the terpolymer is present in a proportion of from 0.01% to 20% by weight of active material, preferably 0.1% to 10% by weight, relative to the total weight of the composition.

3. Composition according to Claim 1 or 2, **characterized in that** the monomer (a) is chosen from C₂-C₆ alkyl acrylates and is preferably ethyl acrylate.

4. Composition according to any one of Claims 1 to 3, **characterized in that** the monomer (b) is chosen from N,N-dimethylaminoethyl methacrylate, N,N-diethylaminoethyl acrylate, N,N-diethylaminoethyl methacrylate, N-t-butylaminoethyl acrylate, N-t-butylaminoethyl methacrylate, N,N-dimethylaminopropylacrylamide, N,N-dimethylaminopropylmethacrylamide, N,N-diethylaminopropylacrylamide and N,N-diethyl-aminopropylmethacrylamide and is preferably N,N-dimethyl-aminoethyl methacrylate.

5. Composition according to any one of Claims 1 to 4, **characterized in that** the monomer (c) is a copolymerizable ethylenic unsaturated surfactant monomer obtained by condensing a nonionic surfactant with itaconic acid.

6. Composition according to any one of Claims 1 to 5, **characterized in that** the acrylic terpolymer consists of acrylates, amino(meth)acrylates and itaconate C₁₀-C₃₀ alkyl, polyoxyethylenated with 20 mol of ethylene oxide.

7. Composition according to any one of Claims 1 to 6, **characterized in that** the acrylic terpolymer also contains a crosslinking monomer.

8. Composition according to any one of Claims 1 to 7, **characterized in that** the polyorganosiloxane with a viscosity of less than 0.1 m².s⁻¹ is chosen from volatile silicones and non-volatile silicones and, in particular:
(i) polyalkylsiloxanes;
(ii) polyarylsiloxanes;
(iii) polyalkylarylsiloxanes;
(iv) organomodified polyorganosiloxanes;
(v) block copolymers containing a polysiloxanepolyalkylene block as repeating unit;
(vi) grafted silicone polymers, containing a non-silicone organic skeleton;
(vii) grafted silicone polymers, containing a polysiloxane skeleton grafted with non-silicone organic monomers;
(viii) and mixtures thereof.

9. Composition according to any one of Claims 1 to 8, **characterized in that** the polyorganosiloxane with a viscosity of less than 0.1 m².s⁻¹ is chosen from polydimethylsiloxanes.

10. Composition according to any one of Claims 1 to 9, **characterized in that** the polyorganosiloxane with a viscosity of less than 0.1 m².s⁻¹ is present in proportions of between 0.01% and 20% by weight and preferably between 0.1% and 10% by weight relative to the total weight of the composition.

11. Composition according to any one of Claims 1 to 10, **characterized in that** the detergent surfactant is chosen from anionic, amphoteric, nonionic and cationic surfactants, and mixtures thereof.

12. Composition according to Claim 11, **characterized in that** the anionic surfactants are chosen from alkaline salts, magnesium salts, ammonium salts, amine salts amino alcohol salts of the following compounds: alkyl sulphates, alkyl ether sulphates, alkylamido ether sulphates, alkylaryl polyether sulphates, monoglyceride sulphates; alkyl sulphonates, alkylamide sulphonates, alkylaryl sulphonates, olefin sulphonates, paraffin sulphonates; alkyl sulphosuccinates, alkyl ether sulphosuccinates, alkylamide sulphosuccinates; alkyl sulphosuccinamates; alkyl sulphoacetates; alkyl phosphates, alkyl ether phosphates; acyl sarcosinates, acyl isethionates and N-acyl taurates; the alkyl or acyl radical in these various compounds generally consists of a carbon-based chain containing from 8 to 30 carbon atoms; fatty acid salts of oleic, ricinoleic, palmitic and stearic acid; coconut oil acid or hydrogenated coconut oil acid; acyl lactylates, in which the acyl radical contains from 8 to 30 carbon atoms; alkyl D-galactosiduronic acids and their salts, polyoxyalkylenated alkyl or alkylaryl ether carboxylic acids or their salts, and polyoxyalkylenated alkylamido ether carboxylic acids or their salts.

13. Composition according to Claim 11, **characterized in that** the nonionic surfactants are chosen from polyethoxylated, polyoxypropylenated or polyglycerolated fatty acids or alkylphenols or alcohols, with a fatty chain containing 8 to 30 carbon atoms, the number of ethylene oxide or propylene oxide groups being between 2 and 50 and the number of glycerol groups being between 2 and 30; copolymers of ethylene oxide and propylene oxide; condensates of ethylene oxide and of propylene oxide with fatty alcohols; polyethoxylated fatty amides; polyglycerolated fatty amides; polyethoxylated fatty amines; oxyethylenated fatty acid esters of sorbitan; fatty acid esters of sucrose, fatty acid esters of polyethylene glycol; alkylpolyglycosides; carbamate or amide derivatives of N-alkylglucamines, aldobionamides and amine oxides.

14. Composition according to Claim 11, **characterized**
**in that** the amphoteric surfactants are chosen from secondary or tertiary aliphatic amine derivatives, in which the aliphatic radical is a linear or branched chain containing 8 to 22 carbon atoms and which contains at least one carboxylate, sulphonate, sulphate, phosphate or phosphonate water-solubilizing anionic group; (C₈-C₂₀)alkylbetaines, sulphobetaines, (C₈-C₂₀)alkylamido(C₁-C₆)alkylbetaines or (C₈-C₂₀)alkylamido(C₁-C₆)alkylsulphobetaines.

15. Composition according to Claim 11, **characterized in that** the cationic surfactants are chosen from salts of optionally polyoxyalkylenated primary, secondary or tertiary fatty amines; quaternary ammonium salts; imidazoline derivatives; and amine oxides of cationic nature.

16. Composition according to any one of Claims 1 to 15, **characterized in that** the detergent surfactant is present in a proportion of at least 4% by weight, preferably from 5% to 50% by weight and even more preferably from 8% to 35% by weight, relative to the total weight of the composition.

17. Composition according to any one of Claims 1 to 16, **characterized in that** the cationic polymer is chosen from:
- proteins or protein hydrolysates, in particular collagen hydrolysates bearing triethylammonium groups, collagen hydrolysates bearing trimethylammonium chloride and trimethylstearylammonium chloride groups, protein hydrolysates bearing on the polypeptide chain quaternary ammonium groups comprising at least one alkyl radical containing from 1 to 18 carbon atoms, and quaternized plant proteins such as wheat, corn or soybean proteins;
- polymers of the polyamine, polyaminoamide or polyquaternary ammonium type, in particular:
i) quaternized or non-quaternized vinylpyrrolidone/dialkylaminoalkyl acrylate or methacrylate copolymers,
ii) cellulose ether derivatives comprising quaternary ammonium groups,
iii) cationic cellulose derivatives such as cellulose copolymers or cellulose derivatives grafted with a water-soluble quaternary ammonium monomer,
iv) polysaccharides and in particular cationic guar gums,
v) polymers consisting of piperazinyl units and of divalent alkylene or hydroxyalkylene radicals containing straight or branched chains, optionally interrupted with oxygen, sulphur or nitrogen atoms or with aromatic or heterocyclic rings, as well as the oxidation and/or quaternization products of these polymers,
vi) water-soluble polyaminoamides prepared in particular by polycondensation of an acidic compound with a polyamine,
vii) polyaminoamide derivatives resulting from the condensation of polyalkylene polyamines with polycarboxylic acids followed by an alkylation with bifunctional agents,
viii) polymers obtained by reacting a polyalkylene polyamine comprising two primary amine groups and at least one secondary amine group with a dicarboxylic acid chosen from diglycolic acid and saturated aliphatic dicarboxylic acids containing from 3 to 8 carbon atoms,
ix) cyclopolymers of methyldiallylamine or of dimethyldiallylammonium,
x) diquaternary ammonium polymers,
xi) polyquaternary ammonium polymers,
xii) homopolymers or copolymers derived from acrylic or methacrylic acids and comprising CH₂-CHRₐ-CO-O-A₁-NRₑR_{f}, CH₂-CHRₐ-CO-O-A₁-N⁺R_{b}R_{c}R_{d}, X⁻ and/or CH₂-CHRₐ-CO-NH-A₁-N⁺R_{b}R_{c}R_{d}, X⁻ units,
in which the groups Rₐ independently denote H or CH₃, the groups A₁ independently denote a linear or branched alkyl group of 1 to 6 carbon atoms or a hydroxyalkyl group of 1 to 4 carbon atoms, the groups R_{b}, R_{c} and R_{d}, which may be identical or different, independently denote an alkyl group of 1 to 18 carbon atoms or a benzyl radical, the groups Rₑ and R_{f} represent a hydrogen atom or an alkyl group of 1 to 6 carbon atoms, X- denotes an anion, for example methosulphate or halide, such as chloride or bromide,
xiii) quaternary polymers of vinylpyrrolidone and of vinylimidazole,
xiv) polyamines referred to under the name "Polyethylene Glycol Tallow Polyamine" in the CTFA dictionary,
xv) crosslinked polymers of methacryloyloxyethyltrimethylammonium salt,
- polyalkyleneimines, polymers containing vinylpyridine or vinylpyridinium units, condensates of polyamines and of epichlorohydrin, polyquaternary ureylenes and chitin derivatives.

18. Composition according to any one of Claims 1 to 17, **characterized in that** the cationic polymer is chosen from cellulose ether derivatives comprising quaternary ammonium groups, cationic guar gums and cyclopolymers of methyldiallylamine or of dimethyldiallylammonium.

19. Composition according to any one of Claims 1 to 18, **characterized in that** it contains a cationic polymer in proportions of between 0.001% and 20% by weight and preferably between 0.05% and 5% by weight relative to the total weight of the composition.

20. Composition according to any one of Claims 1 to 19, **characterized in that** it has a pH of between 3 and 12 and more particularly between 4 and 8.

21. Composition according to any one of Claims 1 to 20, **characterized in that** the cosmetically acceptable medium consists of water, of one or more solvents or of a mixture of water and at least one solvent chosen from lower alcohols, alkylene glycols and polyol ethers.

22. Composition according to any one of Claims 1 to 21, **characterized in that** it also contains at least one silicone resin or gum.

23. Composition according to any one of Claims 1 to 22, **characterized in that** it also contains at least one cosmetically acceptable adjuvant chosen from fragrances, preserving agents, sequestering agents, wetting agents, sugars, plant, animal, mineral or synthetic oils, amphoteric polymers, menthol, nicotinate derivatives, agents for preventing hair loss, antidandruff agents, foam stabilizers, propellants, dyes, screening agents, ceramides, vitamins or provitamins and acidifying or basifying agents.

24. Use, as a shampoo, of the composition as defined in any one of Claims 1 to 23.

25. Process for washing keratin materials, **characterized in that** at least one composition as defined in any one of Claims 1 to 23 is applied to the wet or dry keratin materials and, after an optional period of leaving to stand on these materials, they are rinsed with water.

## Patentansprüche

1. Zusammensetzung zur Reinigung von Keratinsubstanzen, die in einem kosmetisch akzeptablen Medium mindestens einen reinigenden grenzflächenaktiven Stoff, mindestens ein kationisches Polymer und mindestens ein Polyorganosiloxanöl mit einer Viskosität von 0,1 m²·s⁻¹ oder darunter enthält, **dadurch gekennzeichnet, dass** sie ferner mindestens ein Acrylterpolymer enthält, das besteht aus:
- 5 bis 80 Gew.-%, vorzugsweise 15 bis 70 Gew.-% und noch bevorzugter 40 bis 70 Gew.-% eines Acrylatmonomers (a), das unter den C₁₋₆-Alkylacrylaten und C₁₋₆-Alkylmethacrylaten ausgewählt ist;
- 5 bis 80 Gew.-%, vorzugsweise 10 bis 70 Gew.-% und noch bevorzugter 20 bis 60 Gew.-% eines Monomers (b), das unter den heterocyclischen Vinylverbindungen, die mindestens ein Stickstoffatom oder Schwefelatom enthalten, (Meth)acrylamiden, Mono- oder Di-C₁₋₄-alkylamino-C₁₋₄-alkyl(meth)-acrylaten und Mono- oder Di-C₁₋₄-Alkylamino-C₁₋₄-alkyl-(meth)acrylamiden ausgewählt ist;
- 0,1 bis 30 Gew.-% und vorzugsweise 0,1 bis 10 Gew.-% eines Monomers (c), das ausgewählt ist unter:
· Urethanen, die bei der Umsetzung eines monoethylenisch ungesättigten Isocyanats mit einem nichtionischen grenzflächenaktiven Stoff, der ein Sequenzcopolymer von 1,2-Butylenoxid und Ethylenoxid mit endständiger C₁₋₄-Alkoxygruppe einschließt, entsteht;
· copolymerisierbaren, ethylenisch ungesättigten Tensidmonomeren, die bei der Kondensation von nichtionischen grenzflächenaktiven Stoffen mit α,β-ethylenisch ungesättigten Carbonsäuren oder ihren Anhydriden gebildet werden;
· Tensidmonomeren, die unter den Reaktionsprodukten vom Harnstofftyp von monoethylenisch ungesättigten Monoisocyanaten mit nichtionischen grenzflächenaktiven Stoffen, die eine Aminofunktion aufweisen, ausgewählt sind;
· (Meth)allylethern der Formel CH₂=CR₁CH₂OAₘBₙAₚR₂, wobei in der Formel R₁ ein Wasserstoffatom oder die Methylgruppe bedeutet, A eine Propylenoxy- oder Butylenoxygruppe bezeichnet, B Ethylenoxy ist, n Null oder eine ganze Zahl von höchstens 200 und m und p Null oder eine ganze Zahl unter n bedeuten und R₂ eine hydrophobe Gruppe mit mindestens 8 Kohlenstoffatomen ist; und
· nichtionischen Monomeren vom Urethantyp, die bei der Reaktion von nichtionischen grenzflächenaktiven Stoffen mit einer Hydroxygruppe und monoethylenisch ungesättigten Isocyanaten gebildet werden;
wobei die prozentualen Gewichtsanteile der Monomere auf dem Gesamtgewicht der Monomere, die das Terpolymer bilden, basieren.

2. Zusammensetzung nach Anspruch 1, **dadurch gekennzeichnet, dass** das Terpolymer in einer Menge von 0,01 bis 20 Gew.-% Wirkstoff und vorzugsweise 0,1 bis 10 Gew.-%, bezogen auf das Gesamtgewicht der Zusammensetzung, vorliegt.

3. Zusammensetzung nach Anspruch 1 oder 2, **dadurch gekennzeichnet, dass** das Monomer (a) unter den C₂₋₆-Alkylacrylaten ausgewählt ist und es sich vorzugsweise um Ethylacrylat handelt.

4. Zusammensetzung nach einem der Ansprüche 1 bis 3, **dadurch gekennzeichnet, dass** das Monomer (b) unter N,N-Dimethylaminoethylmethacrylat, N,N-Diethylaminoethylacrylat, N,N-Diethylaminoethylmethacrylat, N-*t*-Butylaminoethylacrylat, N-*t*-Butylaminoethylmethacrylat, N,N-Dimethylaminopropylacrylamid, N,N-Dimethylaminopropylmethacrylamid, N,N-Diethylaminopropylacrylamid und N,N-Diethylaminopropylmethacrylamid und vorzugsweise dem N,N-Dimethylaminoethylmethacrylat ausgewählt ist.

5. Zusammensetzung nach einem der Ansprüche 1 bis 4, **dadurch gekennzeichnet, dass** das Monomer (c) ein copolymerisierbares, ethylenisch ungesättigtes Tensidmonomer ist, das durch Kondensation eines nichtionischen grenzflächenaktiven Stoffes mit Itaconsäure entsteht.

6. Zusammensetzung nach einem der Ansprüche 1 bis 5, **dadurch gekennzeichnet, dass** das Acrylterpolymer aus Acrylaten, Amino(meth)acrylaten und mit 20 mol Ethylenoxid polyethoxyliertem C₁₀₋₃₀-Alkylitaconat besteht.

7. Zusammensetzung nach einem der Ansprüche 1 bis 6, **dadurch gekennzeichnet, dass** das Acrylterpolymer ferner ein Monomer zur Vernetzung enthält.

8. Zusammensetzung nach einem der Ansprüche 1 bis 7, **dadurch gekennzeichnet, dass** das Polyorganosiloxan mit einer Viskosität unter 0,1 m²·s⁻¹ unter den flüchtigen Siliconen und den nicht flüchtigen Siliconen und insbesondere den folgenden Verbindungen ausgewählt ist:
(i) Polyalkylsiloxanen;
(ii) Polyarylsiloxanen;
(iii) Polyalkylarylsiloxanen;
(iv) organomodifizierten Polyorganosiloxanen;
(v) Blockcopolymeren mit linearem Polysiloxan-Polyalkylen-Block als wiederkehrende Einheit;
(vi) gepfropften Siliconpolymeren mit nicht siliconhaltigem organischen Grundgerüst;
(vii) gepfropften Siliconpolymeren mit Polysiloxangrundgerüst, auf das nicht siliconhaltige, organische Monomere gepfropft sind; und
(viii) deren Gemische.

9. Zusammensetzung nach einem der Ansprüche 1 bis 8, **dadurch gekennzeichnet, dass** das Polyorganosiloxan mit einer Viskosität unter 0,1 m²·s⁻¹ unter den Polydimethylsiloxanen ausgewählt ist.

10. Zusammensetzung nach einem der Ansprüche 1 bis 9, **dadurch gekennzeichnet, dass** Polyorganosiloxan mit einer Viskosität unter 0,1 m²·s⁻¹ in einer Menge von 0,01 bis 20 Gew.-% und vorzugsweise 0,1 bis 10 Gew.-%, bezogen auf das Gesamtgewicht der Zusammensetzung, enthalten ist.

11. Zusammensetzung nach einem der Ansprüche 1 bis 10, **dadurch gekennzeichnet, dass** der reinigende grenzflächenaktive Stoff unter den anionischen, amphoteren, nichtionischen und kationischen grenzflächenaktiven Stoffen und deren Gemischen ausgewählt ist.

12. Zusammensetzung nach Anspruch 11, **dadurch gekennzeichnet, dass** die anionischen grenzflächenaktiven Stoffe unter den Alkalisalzen, Magnesiumsalzen, Ammoniumsalzen, Aminsalzen oder Aminoalkoholsalzen der folgenden Verbindungen: Alkylsulfate, Alkylethersulfate, Alkylamidoethersulfate, Alkylarylpolyethersulfate, Monoglyceridsulfate; Alkylsulfonate, Alkylamidsulfonate, Alkylarylsulfonate, Olefinsulfonate, Paraffinsulfonate; Alkylsulfosuccinate, Alkylethersulfosuccinate, Alkylamidsulfosuccinate; Alkylsulfosuccinamate; Alkylsulfoacetate; Alkylphosphate, Alkyletherphosphate; Acylsarcosinate, Acylisethionate und N-Acyltaurate; wobei die Alkyl- oder Acylgruppen dieser verschiedenen Verbindungen aus einer Kohlenstoffkette mit 8 bis 30 Kohlenstoffatomen bestehen; Salzen von Ölsäure, Ricinolsäure, Palmitinsäure und Stearinsäure; Säuren von Kopraöl oder hydriertem Kopraöl; Acyllactylaten, deren Acylgruppe 8 bis 30 Kohlenstoffatome aufweist; Alkyl-D-galactosiduronsäuren und ihren Salzen, polyalkoxylierten Alkyl- oder Carbonsäurealkylarylethern oder ihren Salzen und polyalkoxylierten Carbonsäurealkylamidoethern oder ihren Salzen ausgewählt sind.

13. Zusammensetzung nach Anspruch 11, **dadurch gekennzeichnet, dass** die nichtionischen grenzflächenaktiven Stoffe unter den Alkoholen oder Alkylphenolen oder Fettsäuren, die polyethoxyliert, polypropoxyliert oder mehrfach mit Glycerin verethert sind und eine Fettkette mit 8 bis 30 Kohlenstoffatomen aufweisen, wobei die Zahl der Ethylenoxid- und Propylenoxidgruppen im Bereich von 2 bis 50 und die Anzahl der Glyceringruppen im Bereich von 2 bis 30 liegt; den Copolymeren von Ethylenoxid und Propylenoxid; den Kondensaten von Ethylenoxid und Propylenoxid mit Fettalkoholen; polyethoxylierten Fettamiden; mehrfach mit Glycerin veretherten Fettamiden; polyethoxylierten Fettaminen; ethoxylierten Sorbitanfettsäureestern; Saccharosefettsäureestem oder Polyethylenglykolfettsäureestern; Alkylpolyglykosiden; Amid- oder Carbamatderivaten von N-Alkylglucaminen, Aldobionamiden und Aminoxiden ausgewählt sind.

14. Zusammensetzung nach Anspruch 11, **dadurch gekennzeichnet, dass** die amphoteren grenzflächenaktiven Stoffe unter den aliphatischen, sekundären oder tertiären Aminderivaten, wobei die aliphatische Gruppe eine lineare oder verzweigte Kette mit 8 bis 22 Kohlenstoffatomen bedeutet, die mindestens eine wasserlösliche anionische Gruppe, Carboxylat, Sulfonat, Sulfat, Phosphat oder Phosphonat aufweist; C₈₋₂₀-Alkylbetainen; Sulfobetainen, C₈₋₂₀-Alkyl-C₁₋₆-amidoalkylbetainen oder C₈₋₂₀-Alkyl-C₁₋₆amidoalkylsulfobetainen ausgewählt sind.

15. Zusammensetzung nach Anspruch 11, **dadurch gekennzeichnet, dass** die kationischen grenzflächenaktiven Stoffe unter den primären, sekundären oder tertiären, gegebenenfalls polyalkoxylierten Aminsalzen; quartären Ammoniumsalzen; Imidazolinderivaten; und Aminoxiden mit kationischen Charakter ausgewählt sind.

16. Zusammensetzung nach einem der Ansprüche 1 bis 15, **dadurch gekennzeichnet, dass** der reinigende grenzflächenaktive Stoff in einer Menge von mindestens 4 Gew.-%, vorzugsweise 5 bis 50 Gew.-% und besonders bevorzugt 8 bis 35 Gew.-%, bezogen auf das Gesamtgewicht der Zusammensetzung, vorliegt.

17. Zusammensetzung nach einem der Ansprüche 1 bis 16, **dadurch gekennzeichnet, dass** das kationische Polymer ausgewählt ist unter:
- Proteinen oder Proteinhydrolysaten, insbesondere Kollagenhydrolysaten mit Triethylammoniumgruppen, Kollagenhydrolysaten mit Trimethylammoniumchlorid- und Trimethylstearylammoniumchloridgruppen, Proteinhydrolysaten, die an der Polypeptidkette quartäre Ammoniumgruppen tragen, die mindestens eine C₁₋₁₈-Alkylgruppe aufweisen, und quaternisierten pflanzlichen Proteinen, wie Weizenproteinen, Maisproteinen oder Sojaproteinen;
- Polymeren vom Polyamintyp, Polyaminoamidtyp oder quartären Polyammoniumtyp, insbesondere
(i) Copolymeren von Vinylpyrrolidon und Dialkylaminoalkylacrylat oder Dialkylaminoalkylmethacrylat, die gegebenenfalls quatemisiert sind,
(ii) Derivaten von Celluloseethern, die quartäre Ammoniumgruppen enthalten,
(iii) kationischen Cellulosederivaten, wie Cellulosecopolymere oder Cellulosederivate, die mit einem wasserlöslichen quartären Ammoniummonomer gepfropft sind,
(iv) Polysacchariden und insbesondere kationischen Guargummen,
(v) Polymeren, die aus Piperazinyleinheiten und zweiwertigen Alkylen- oder Hydroxyalkylengruppen mit geraden oder verzweigten Ketten bestehen, die gegebenenfalls mit Sauerstoffatomen, Schwefelatomen, Stickstoffatomen oder aromatischen oder heterocyclischen Ringen unterbrochen sind, sowie den Oxidationsprodukten und/oder Quaternisierungsprodukten dieser Polymere,
(vi) wasserlöslichen Polyaminoamiden, die insbesondere durch Polykondensation einer sauren Verbindung mit einem Polyamin hergestellt werden,
(vii) Derivaten von Polyaminoamiden, die bei der Kondensation von Polyalkylenpolyaminen mit Polycarbonsäuren und anschließender Alkylierung mit bifunktionellen Mitteln entstehen,
(viii) Polymeren, die durch Umsetzung eines Polyalkylenpolyamins, das zwei primäre Aminogruppen und mindestens eine sekundäre Aminogruppe aufweist, mit einer Dicarbonsäure hergestellt werden, welche unter Diglykolsäure und den gesättigten, aliphatischen Dicarbonsäuren mit 3 bis 8 Kohlenstoffatomen ausgewählt ist,
(ix) Methyldiallylamin- oder Dimethyldiallylammonium-Cyclopolymeren,
(x) quartären Diammoniumpolymeren,
(xi) quartären Polyammoniumpolymeren,
(xii) Homo- oder Copolymeren, die von Acrylsäure oder Methacrylsäure abgeleitet sind und die Einheiten CH₂-CHRₐ-CO-O-A₁-NRₑR_{f}, CH₂-CHRₐ-CO-O-A₁-N⁺R_{b}R_{c}R_{d}X⁻ und/ oder CH₂-CHRₐ-CO-NH-A₁-N⁺R_{b}R_{c}R_{d}X⁻ enthalten, wobei die Gruppen Rₐ unabhängig H oder CH₃ bedeuten, die Gruppen A₁ unabhängig voneinander eine geradkettige oder verzweigte Alkylgruppe mit 1 bis 6 Kohlenstoffatomen oder eine Hydroxyalkylgruppe mit 1 bis 4 Kohlenstoffatomen bedeuten, die Gruppen R_{b}, R_{c} und R_{d}, die identisch oder voneinander verschieden sind, unabhängig eine Alkylgruppe mit 1 bis 18 Kohlenstoffatomen oder Benzyl bedeuten, die Gruppen Rₑ und R_{f} Wasserstoff oder eine Alkylgruppe mit 1 bis 6 Kohlenstoffatomen bedeuten und X- ein Anion ist, beispielsweise Methosulfat oder ein Halogenid, wie Chlorid oder Bromid,
(xiii) quartären Polymeren von Vinylpyrrolidon und Vinylimidazol,
(xiv) Polyaminen, die nach CTFA-Nomenklatur als "POLYETHYLENEGLYCOL TALLOW POLYAMINE" bezeichnet werden, und
(xv) vernetzten Polymeren von Methacryloyloxyethyltrimethylammoniumsalzen,
- Polyalkyleniminen, Polymeren, die Vinylpyridin- oder Vinylpyridinium-Einheiten enthalten, Kondensaten von Polyaminen und Epichlorhydrin, quartären Polyureylenen und Chitinderivaten.

18. Zusammensetzung nach einem der Ansprüche 1 bis 17, **dadurch gekennzeichnet, dass** das kationische Polymer unter den Celluloseetherderivaten mit quartären Ammoniumgruppen, den kationischen Guargummen und den Methyldiallylamin-Cyclopolymeren oder Dimethyldiallylammonium-Cyclopolymeren ausgewählt ist.

19. Zusammensetzung nach einem der Ansprüche 1 bis 18, **dadurch gekennzeichnet, dass** sie ein kationisches Polymer in Mengenanteilen von 0,001 bis 20 Gew.-% und vorzugsweise 0,05 bis 5 Gew.-%, bezogen auf das Gesamtgewicht der Zusammensetzung, enthält.

20. Zusammensetzung nach einem der Ansprüche 1 bis 19, **dadurch gekennzeichnet, dass** sie einen pH-Wert im Bereich von 3 bis 12 und insbesondere 4 bis 8 aufweist.

21. Zusammensetzung nach einem der Ansprüche 1 bis 20, **dadurch gekennzeichnet, dass** das kosmetisch akzeptable Medium aus Wasser oder einem oder mehreren Lösungsmitteln oder einem Gemisch von Wasser und mindestens einem Lösungsmittel besteht, das unter den niederen Alkoholen, Alkylenglykolen und Polyolethern ausgewählt ist.

22. Zusammensetzung nach einem der Ansprüche 1 bis 21, **dadurch gekennzeichnet, dass** sie ferner mindestens ein Siliconharz oder mindestens einen Silicongummi enthält.

23. Zusammensetzung nach einem der Ansprüche 1 bis 22, **dadurch gekennzeichnet, dass** sie außerdem mindestens einen kosmetisch akzeptablen Zusatzstoff enthält, der ausgewählt ist unter: Parfums, Konservierungsmitteln, Maskierungsmitteln, Feuchthaltemitteln, Zuckern, pflanzlichen, mineralischen, tierischen oder synthetischen Ölen, amphoteren Polymeren, Menthol, Nicotinatderivaten, Wirkstoffen gegen Haarausfall, Wirkstoffen gegen Schuppen, Schaumstabilisatoren, Treibmitteln, Filtern, Farbmitteln, Ceramiden, Vitaminen oder Provitaminen und Ansäuerungs- oder Alkalisierungsmitteln.

24. Verwendung der Zusammensetzung nach einem der Ansprüche 1 bis 23 als Haarwaschmittel.

25. Verfahren zur Reinigung von Keratinsubstanzen, **dadurch gekennzeichnet, dass** auf die feuchten oder trockenen Keratinsubstanzen mindestens eine Zusammensetzung nach einem der Ansprüche 1 bis 23 aufgetragen und nach einer fakultativen Einwirkzeit mit Wasser gespült wird.
